# EUROPEAN PATENT APPLICATION

(11) **EP 2 422 776 A1**
(43) Date of publication of application: **29.02.2012**
(21) Application number: 10766686.9
(22) Date of filing: 22.04.2010
(51) Int. Cl.: A61K 9/51, A61K 31/337, A61P 35/00

(54) **POLYARGININE NANOCAPSULES**

(30) Priority: 22.04.2009 ES 200901098
(71) Applicant: Universidade De Santiago De Compostela, 15782 Santiago de Compostela (ES)
(72) Inventor: LOZANO LOPEZ, Victoria, (ES); ALONSO FERNANDEZ, Maria Jose, (ES); TORRES LOPEZ, Dolores, (ES)
(74) Representative: ABG Patentes, S.L.
(86) International application number: PCT/ES2010/070247
(87) International publication number: WO 2010/122204

(57) **Abstract**

The present invention relates to a system for administering active ingredients comprising nanocapsules with a diameter less than 1 µm comprising a polyarginine salt, a negative phospholipid and an oil. The invention also relates to methods for obtaining said nanocapsule system, the pharmaceutical and cosmetic compositions thereof, as well as the use thereof in medicine, particularly in the preparation of a drug for treating cancer.

## Description

### Technical Field

The present invention relates to a system for administering active ingredients comprising nanometric-sized nanocapsules, said active ingredients are both hydrophilic and hydrophobic, as well as to the pharmaceutical and cosmetic compositions comprising the same and methods for preparing them.

### Background of the Invention

The physicochemical properties of the active ingredients are determining characteristics in the pharmaceutical development of these molecules which can further determine their therapeutic efficacy. More specifically, the low solubility of many active ingredients in water complicates their formulation often requiring the use of excipients which can cause serious side effects as well as complications in the treatment. In addition, the large hydrophilic molecules have difficulties in passing biological barriers and their stability by degradation is compromised due to the defense mechanisms of the organism. These difficulties must be solved so that the active molecules arrive at the therapeutic target and thus achieve an effective therapy.

The incorporation of active ingredients in nanometric-sized systems has helped to improve the formulation limitations of these molecules, additionally increasing their therapeutic potential. Solubility improvement, protection against degradation or greater penetration of the active ingredients, are some of the advantages which the nanoencapsulation of the active molecule offers.

It is also known that the ability of these systems to cross external barriers and gain access into the organism depends on both the size and composition thereof. Small particles will increase the degree of transport with respect to those of a larger size: nanosystems having diameter less than 1 µm meet this criterion. If they are prepared from natural, biocompatible and biodegradable polymers, the possibilities that the nanosystems are naturally transported through the mucosae of the organism by means of known transport mechanisms and without altering the physiology of the epithelium are increased.

Lecithin is a natural substance the main component of which is phosphatidylcholine (neutral phospholipid) and the secondary components of which are phosphatidylinositol, phosphatidic acid (negatively charged phospholipids), other phospholipids such as phosphatidylethanolamine and lysophosphatidylcholine, and other components such as tocopherol or triglycerides.

Polyarginine (PArg) is a hydrophilic cationic polyamino acid which maintains its positive charge in almost the entire pH range due to the strong alkaline character of its guanidine groups. This homopolymer is included within the family of those so-called protein transduction domains (PTD). PTDs are molecules which have shown their capacity to effectively crossing biological membranes and have been used to increase the cellular uptake of proteins and of larger molecules after their chemical modification. Therefore, the clinical studies carried out for obtaining a hepatitis C vaccine made up of polyarginine polyamino acid as an immunostimulant show the increase of the immune response by the T cells without observing antigenicity by the polymer (Mattner et al. Cancer Res. (2002) 62:1477). This internalization capacity has also been used in gene therapy in the modification of liposomes for carrying siRNA high transfection levels being obtained (Zhang et al. J. Control. Release. (2006) 112(2):229).

Different studies have also demonstrate the promoter capacity for absorbing polyarginine for the passage of fluorescent markers through the nasal mucosae (Miyamoto et al. Eur. J. Pharm. Biopharm (2001) 52(1):21), of the atrial natriuretic peptide or calcitonin (Natsume et al. Drug Deliv. Syst. (1999) 14:21). Document US2003215491 also describes the polyarginine capacity as a promoter for absorbing bioactive molecules through the nose by means of the formation of lipid matrices made up of phospholipids or oils.

Document US005716614A1 also uses the combination of fatty acids with polyarginine for the formation of active molecule release systems such as micelles, vesicles or liposomes directed at the central nervous system.

Other systems formulated from polyarginine are disclosed in documents US2006269606 and W02006116546 in which the formation of microcapsules formed by a core coated by polyarginine polyamino acid for carrying active molecules is described. At this time it is convenient to point out that the microencapsulation techniques intended for forming microparticles or microcapsules generally differ from nanotechnology techniques applied for forming nanosystems. Likewise, the colloidal systems obtained by both processes have very different characteristics not only in relation to their size, but also with respect to the behavior thereof and subsequent application.

In addition, arginine is widely known as nitric oxide precursor, an important vasodilator agent; therefore polyarginine has been used as therapeutic agent for coating cardiovascular implants. Likewise, document US2006062821 describes the formulation of polymer cores coated with polyarginine for obtaining liposomes, microspheres or coated emulsions intended for forming implantable devices in cardiology.

Therefore there is a growing need of providing alternative active ingredients release systems to those currently existing. Particularly, it would be convenient to provide stable nanosystems for certain applications which were suitable for encapsulating molecules of different solubility and which will further have good internalization and adsorption properties on the biological surfaces.

### Description of the Invention

The authors of the present invention have developed an easy-to-obtain nanocapsule system by means of different experimental methods, wherein the nanocapsules comprise polyarginine in the form of salt, and an oily core which in turn comprises an oil and a negative phospholipid component. Said nanocapsule systems allow an efficient association of active ingredients regardless of their hydrophilicity/hydrophobicity, and the subsequent release in a suitable medium. The reduced size of said nanocapsules, diameter less than 1µm, enables their passage through mucosae and that they are internalized by the cells. Likewise, the presence of a polyarginine coating facilitates their absorption and grants stability to the nanocapsules, in addition to conferring a highly positive surface charge which allows the interaction with biological surfaces of the negatively charged organism, such as mucosae or tumor cells.

Thus a first aspect of the invention relates to a system for administering active ingredients comprising nanocapsules with a diameter less than 1 µm comprising a polyarginine salt, a negative phospholipid and an oil.

In another aspect, the invention relates to a pharmaceutical composition comprising the systems defined above.

In another aspect, the invention relates to a cosmetic composition comprising the systems defined above.

Likewise, the invention relates to the use of said systems in the preparation of a medicinal product. In a particular embodiment, said use relates to cancer treatment.

In an additional aspect, the invention relates to a method for obtaining the systems defined above, which comprises:
a) preparing an aqueous solution of a polyarginine salt;
b) preparing an organic solution of a negative phospholipid and an oil;
c) mixing the solutions prepared in steps a) and b) under stirring, the nanocapsules being spontaneously obtained; and
d) optionally, completely or partially evaporating the organic solvents from the mixture obtained in the preceding step to a constant volume.

According to particular embodiments, an active ingredient is encapsulated by addition to step a) or to step b) or even once the nanocapsules are formed, depending on the solubility of said active ingredient.

In a more particular embodiment, a hydrophobic active ingredient is added to the organic solution of step b). In another more particular embodiment, if the active ingredient is hydrophilic it is preferably added dissolved in water in step b). According to another particular embodiment, a hydrophilic active ingredient can be incorporated by means of incorporating the same to the nanocapsules obtained in step d).

In another additional aspect, the invention relates to a method for obtaining the systems defined above, which comprises coating a nanoemulsion made up of at least a negative phospholipid, an oil and an aqueous phase, with a polyarginine salt by means of an incubation process with an aqueous solution of a polyarginine salt.

According to a particular embodiment, the method further comprises adding an active ingredient.

According to a more particular embodiment, if the active ingredient is hydrophilic, said active ingredient is added to the nanoemulsion, preferably dissolved in water. According to another more particular embodiment, if the active ingredient is hydrophobic, said active ingredient is added in the process of forming the nanoemulsion, preferably dissolved in ethanol.

According to another particular embodiment, the active ingredient is incorporated by means of adsorption to the nanocapsules previously formed.

According to particular embodiments of the previous methods, an auxiliary component is added to the solution prepared in step b) or to the nanoemulsion, which will preferably be a hydrophobic polyoxyethylene derivative, and more preferably polyethylene glycol stearate. This auxiliary component allows modulating the surface electrical charge of the nanocapsules of the system, as well as providing them a protective shield to improve their stability in biological fluids.

### Description of the Drawings

Figure 1 shows the TEM images of polyarginine (PArg) nanocapsule systems at 3 and at 6% w/w, respectively.
Figure 2 shows the evaluation of the degree of pDNA association to the polyarginine nanocapsule systems (NCs) by means of electrophoresis at different charge proportions (3, 10 and 300). The systems studied were:
   - free pDNA
   - Polyarginine/pDNA nanocapsule systems
   - Polyarginine/pDNA nanocapsules systems + heparin
Figure 3 shows the size of the DNA/polyarginine complexes in water and in phosphate buffer.
Figure 4 shows the evaluation of the degree of pDNA association to the pDNA/polyarginine nanocapsule systems, to the pDNA/polyarginine-PEG nanocapsule systems and to the pDNA/polyarginine complexes.
Figure 5 shows the docetaxel (DCX) release profile of polyarginine nanocapsule systems.
Figure 6 shows the evaluation of the particle size of the 3% w/w polyarginine nanocapsule system formulation during their storage at 4°C and 37°C (size (nm) vs. time (months))
Figure 7 shows the evaluation of the particle size of the 6% w/w polyarginine nanocapsule system formulation during their storage at 4°C and 37°C (size (nm) vs. time (months))
Figure 8 shows the evaluation of the trehalose cryoprotective effect (at 5 and at 10% w/v) on the particle size of the polyarginine nanocapsule systems (at 3 and at 6% w/w) after the lyophilization process.
Figure 9 shows the polyarginine nanocapsule system cell proliferation inhibition capacity study. The % of cell viability at different concentrations of DCX is depicted for polyarginine NC, DCX in solution, polyarginine/DCX NC.
Figure 10 shows the polyarginine nanocapsules system cellular uptake study. Cell entry values obtained from the polyarginine nanocapsule systems in comparison with the nanoemulsion and the free fluorescent marker.
Figure 11 shows the polyarginine nanocapsule system cellular uptake study depending on the amount of polyarginine in the nanosystem. Cell entry values obtained from the 3 and 6% w/w polyarginine nanocapsule system formulation.

### Detailed Description of the Invention

The present invention relates to the preparation of nanocapsule systems for administering active ingredients, wherein the nanocapsules of the system have a diameter less than 1 µm and comprise a polyarginine salt, a negative phospholipid, and an oil.

The electrostatic interaction which is produced between the positively protonated form of the polyarginine with the negative phospholipid forms the nanocapsule systems. It is important to point out the difference existing between the nanocapsule systems and nanoemulsion systems, since the advantage of the nanocapsule systems with respect to the nanoemulsion systems is the presence of a polymer coating the oily cores of the nanocapsules which can confer to them mucoadhesive property, absorption-promoting property or protection against aggregation. These nanocapsule systems will further have advantages with respect to other larger systems (microparticles, pellets, films, sponges...) in terms of their biological applications. In fact, it is known that the interaction of a drug release system with a biological surface is highly conditioned by its size. Thus the nanocapsules are able of crossing mucosae and being internalized by the cells acting as drug transport systems whereas the microparticles do not have that capacity. Similarly the biodistribution of these systems is highly conditioned by the size. The knowledge of the drug release colloidal systems generated in last few years worldwide has allowed fixing a clearly defined border between nanosystems (less than one micron) and microparticle systems.

Likewise it is important to point out the difference between nanocapsule systems and "complexes". "Complexes" is understood as the nanostructure formed by the interaction of polyelectrolytes or by polyelectrolytes and surfactant of opposite charge. The nanocapsule systems of the present invention are different from polyarginine complexes (Kim et al. Mol. Ther. (2006) 14:343) since they relate to a nanocapsular transport system providing greater homogeneity to the system, as well as the possibility of co-encapsulating other components in the core like such as example active ingredients or excipients. These characteristics allow maintaining the integrity and functionality of the nanostructure as well as providing greater stability in the presence of biological fluids.

The nanocapsules of the systems of the present invention have a diameter less than 1 µm, therefore conforming to the definition of a nanosystem, colloidal system made up of a polymer base with a size less than 1 µm, i.e., they have a size between 1 and 999 nm, preferably between 50 and 500 nm. The size of the nanocapsules is mainly influenced by the formation conditions and composition and can be measured using standard methods known by the person skilled in the art which are described, for example, in the experimental part below. The size thereof does not notably vary upon modifying the polyarginine ratio in the formulation, nanometric-sized systems being obtained in any case.

The surface charge of the polyarginine nanocapsules have highly positive values, it being an important aspect since it is often of interest for obtaining greater interaction with the biological surfaces of the organism, and particularly with the mucosae or with tumor cells, the surface of which is more negative in relation to the healthy cells. Therefore, the positive charge of the nanocapsules favors the interaction with biological surfaces and as a consequence it the active ingredients associated to the polyarginine nanocapsule system acting on the target tissues will be favored.

In a particular embodiment, the polyarginine salt is selected from hydrochloride, hydrobromide, acetate and sulfate. Preferably the polyarginine salt is hydrochloride.

In the present invention, the term "negative phospholipid" refers to a phospholipid component comprising a single negatively charged phospholipid or a mixture of phospholipid the total charge of which is negative, as is the case of some naturally occurring phospholipids, such as lecithin. In a particular embodiment, the negative phospholipid is selected from lecithin, phosphatidylglycerol, phosphatidylserine, phosphatidylinositol, diphosphatidylglycerol and phosphatidic acid. Preferably the negative phospholipid is lecithin.

In another particular embodiment, the oil is selected from peanut oil, cotton oil, olive oil, castor oil, soybean oil, safflower oil, palm oil; vitamin E, isopropyl myristate, squalene, Mygliol®, Labrafil®, Labrafac®, Peceol® and Maisine®. Preferably the oil is Mygliol®.

The term "active ingredient" refers to any substance which is used in treating, curing, preventing or diagnosing a disease or which is used for improving physical and mental wellbeing of human beings and animals. The active ingredient can be for example a drug, a vitamin, a vaccine, etc., or a cosmetic agent. The nanocapsule systems object of the present invention is suitable for incorporating active ingredients regardless of the solubility characteristics thereof. The association capacity will depend on the molecule incorporated, but in general terms will be elevated both for hydrophilic molecules and for very hydrophobic molecules. In a particular embodiment, the active ingredient is selected from peptides, proteins, lipid or lipophilic compounds, saccharide compounds, compounds of nucleic acids or nucleotides such as oligonucleotides, polynucleotides or combinations of the molecules mentioned.

In a preferred embodiment, the active ingredient is docetaxel. In another preferred embodiment, the active ingredient is selected from a nucleic acid, such as an oligonucleotide, interference RNA, a DNA plasmid or a polynucleotide, preferably the active ingredient is a DNA plasmid.

The proportion of active ingredient incorporated in the system can be up to approximately 50% by weight with respect to the total weight of the components of the system. However, in each case the suitable proportion will depend on the active ingredient to be incorporated, the indication for what it is used and the administration efficiency. In a particular embodiment, the proportion of active ingredient can be up to approximately 10% by weight, preferably up to approximately 5%.

In the specific case of incorporating a polynucleotide such as a DNA plasmid or interference RNA as an active ingredient, the proportion thereof in said system can be up to approximately 30% by weight, preferably up to approximately 10%.

In a particular embodiment, the systems defined above can incorporate in their capsular structure an auxiliary component which improves their stability in biological fluids and reduces their uptake by the immune system (mononuclear phagocyte system). This auxiliary component will preferably be a hydrophobic polyoxyethylene derivative, and more preferably polyethylene glycol stearate.

The method for obtaining the nanocapsule systems is a simple method which prevents drastic conditions such as high temperatures. Carrying out any type of chemical reaction for obtaining the same is also unnecessary since as has been indicated, the method for obtaining the system is by means of ionic interaction. Therefore, the integrity of the molecules incorporated into the system, which are susceptible to degradation, is thus preserved. To form nanocapsules in a desired size range, the oily cores made up of the oil and coated by the negative phospholipid is formed; the electrostatic interaction between the phospholipid and the polyarginine polyamino acid which will lead to obtaining the polyarginine nanocapsules is simultaneously produced. It is therefore a solvent emulsification-diffusion process which occurs in a controlled manner and will provide stability to the system, without the need of creating covalent bonds between the components.

A particular method for obtaining the systems of the invention comprises:
a) preparing an aqueous solution of a polyarginine salt;
b) preparing an organic solution of a negative phospholipid and an oil;
c) mixing the solutions prepared in steps a) and b) under stirring, the nanocapsules being spontaneously obtained; and
d) optionally, completely or partially evaporating the organic solvents from the mixture obtained in the preceding step to a constant volume.

The systems of the present invention being able to be prepared by means of an alternative method which comprises coating a nanoemulsion with a polyarginine salt by means of an incubation process with an aqueous solution of the polymer.

In a particular embodiment the incubation process comprises mixing the nanoemulsion with an aqueous solution of polyarginine. In a more particular embodiment, the incubation process comprises mixing the nanoemulsion with an aqueous solution of polyarginine in a preferred 4:1 (nanoemulsion:solution of PArg) ratio, the coating being immediately produced.

Said nanoemulsion is made up of at least a negative phospholipid, an oil and an aqueous phase. The aqueous phase is preferably bidistilled water, although it may contain surfactants, salts, and other auxiliary agents.

The methods for preparing said nanoemulsion are known in the state of art and may comprise a diffusion, sonication or homogenization process (Prego et al. J. Nanosci. Nanotechnol. (2006) 6:1; Tadros et al. Adv. Colloid Interface Sci. (2004) 109:303).

A particular method for obtaining the nanoemulsion comprises:
i) preparing an organic solution of a negative phospholipid and an oil;
ii) adding the solution obtained in step i) on an aqueous phase under stirring to form a nanoemulsion;
iii) optionally, completely or partially evaporating the organic solvents to a constant volume.

Another particular method for obtaining the nanoemulsion comprises:
i) preparing an organic solution of a negative phospholipid and an oil;
ii) adding the solution obtained in step i) on an aqueous phase and sonicating for approximately 1 minute;
iii) diluting approximately 1:10 the emulsion obtained in the phase ii) with water
iv) optionally, completely or partially evaporating the organic solvents to a constant volume.

Another particular method for obtaining the nanoemulsion comprises:
i) preparing an organic solution of a negative phospholipid and an oil;
ii) adding the solution obtained in step i) on an aqueous phase and homogenizing for approximately 5 minutes;
iii) diluting approximately 1:8 the emulsion obtained in phase ii) with water and homogenizing for approximately 10 minutes;
iv) optionally, completely or partially evaporating the organic solvents to a constant volume.

According to a particular embodiment of the previous methods, an auxiliary component made up of a hydrophobic polyoxyethylene derivative is added to the solution of step b) or to step i). Preferably, the component is polyethylene glycol stearate. In a more preferred embodiment, the polyethylene glycol stearate is in an approximate proportion between 1 and 2% w/v (weight of polyethylene glycol stearate/volume of formulation).

According to particular embodiments of the previous methods, if the active ingredient is hydrophobic, said active ingredient is added to the organic solution of step b) or of step i). Preferably, said hydrophobic active ingredient is added dissolved in ethanol. According to other particular embodiments, if the active ingredient is hydrophilic, said active ingredient is added to the solution of step b) or of step i). Preferably, said hydrophilic active ingredient is added dissolved in an aqueous solution. It is also possible to incorporate it by means of adsorption to the nanocapsule suspension obtained in step d) or after the incubation process once the nanocapsules are formed.

The nanocapsules are formed upon mixing volumes of the mentioned solutions in different ratios. Thus, the nanocapsules will have a composition constant with respect to the oily cores, varying the proportion of polyarginine between approximately 1 and 27% w/w (weight of polyarginine/weight of formulation). Preferably, the aqueous solution of a polyarginine salt is formed by 0.1-60 mg of polyarginine.

The solvent of the organic solution is preferably a mixture of ethanol/acetone, yet more preferably in an approximate ratio between 1/15 and 1/20, or other organic solvents such as dichloromethane. In a particular embodiment, the oil is added on the phospholipid solution.

A particular example for obtaining the nanocapsule systems of the invention following the first method described above comprises:
a) preparing an aqueous solution of a polyarginine salt of 10 ml in volume in which 1 to 30 mg of polyarginine is dissolved;
b) preparing an oily phase formed by an ethanol/acetone (0.25:4.7 ml) solution of lecithin (20 mg) and optionally PEG stearate (48-96 mg), to which 62.5 µl of Miglyol® 812 is added. A hydrophilic active ingredient dissolved in a small volume of aqueous phase (25 µl) can be incorporated in this step, if desired;
c) mixing under stirring the solutions resulting from steps a) and b), the nanocapsules being spontaneously obtained;
d) optionally, evaporating the organic solvents of the mixture obtained in the previous step to a constant volume.

The method for manufacturing the polyarginine nanocapsule systems can include an additional lyophilization step in order to preserve them during their storage so that their initial characteristics are conserved. To lyophilize the systems the addition of sugars exerting cryoprotective effect is necessary. Among the sugars useful for carrying out the lyophilization are, for example, trehalose, glucose, sucrose, mannitol, maltose, polyvinyl pyrrolidone (PVP). In the lyophilized form, the nanocapsules can be stored for long time periods and can be easily regenerated, if necessary, by simply adding an optimum volume of water.

According to this additional step, the present invention also relates to the polyarginine nanocapsule systems in the lyophilized form.

The nanocapsule systems described herein have a suitable stability both in suspension and in the lyophilized form, therefore they can be stored for long time periods. In addition, their stability in determined biological fluids which assures they will remain in nanocapsular form after administration to organisms, human or animal, has also been studied.

The polyarginine nanocapsule systems of this invention have advantages in comparison with other systems for administering and/or releasing drugs since they have improved stability in biological fluids in comparison with other nanosystems, such as for example in comparison with complexes. The encapsulation of active ingredients, such as pDNA, in the polyarginine nanocapsules described herein, is preferable in comparison with the pDNA/polyarginine complexes due to the improvement in the stability of the system. Furthermore, the existence of a nanocapsular carrier provides a greater homogeneity to the system, as well the possibility of co-encapsulating other components, such as active ingredients or excipients, in the core of the nanocapsule.

The nanocapsule systems of the invention comprising polyarginine in their composition have demonstrated to have excellent absorption-promoting properties due to the great cell-internalization capacity which said polyamino acid has, converting them into systems very useful as pharmaceutical and cosmetic systems.

Thus, the invention in a particular embodiment relates to a pharmaceutical composition, comprising the nanocapsule systems above, and optionally one or more pharmaceutically acceptable excipients. In particular, the incorporation of active ingredients in the nanocapsules of the invention gives rise to systems, the determined characteristics in terms of their composition, properties and morphology of which confer them with a large potential in the therapeutic field. The active ingredient to be incorporated in the systems of the invention will be that with suitable pharmacotherapeutic properties according to the therapeutic application for which the formulation is intended. In a particular embodiment, the active ingredient is selected from peptides, proteins, lipid or lipophilic compounds, saccharide compounds, compounds of nucleic acids and nucleotides such as oligonucleotides, polynucleotides or combinations of the mentioned molecules

In a preferred embodiment, the active ingredient is docetaxel. In another preferred embodiment, the active ingredient is selected from a nucleic acid, such as a oligonucleotide, interference RNA, a DNA plasmid or a polynucleotide, preferably the active ingredient is a DNA plasmid.

Said pharmaceutical compositions can be administered through different routes, such as through mucosae, topically or parenterally, and among them the administration through mucosae is of most interest.

The ratio of active ingredient incorporated in the systems can be up to approximately 50% by weight with respect to the total weight of the components of the nanocapsule system. However in each case, the suitable ratio will depend on the active ingredient to be incorporated, the indication for what it is used and the administration efficiency. In a particular embodiment, the ratio of active ingredient can be up to approximately 10% by weight, preferably up to approximately 5%.

In the specific case of incorporating a polynucleotide such as a DNA plasmid or interference RNA as active ingredient, the ratio thereof in said system may reach up to approximately 30% by weight, preferably up to approximately 10%.

Due to its good properties for administering on or through the skin, and to its lasting stability, the nanocapsule system of the invention is also suitable for cosmetic applications. Therefore, in another aspect, the invention relates to a cosmetic composition comprising the nanocapsule systems described above together with one or more cosmetically acceptable excipients. The cosmetic compositions according to the invention include any liquid composition (nanocapsule suspension or dispersion) or any composition in the form of gel, cream, ointment or balm for topical administration. Said cosmetic composition can be applied to various surface parts of the human or animal body such as the skin, hair system, nails, lips and external genitalia, and to teeth or mucosae of the human or animal body. In a particular embodiment of the invention, the cosmetic composition has a hygienic and aesthetic purpose, or is used to neutralize or remove ectoparasites, or for the purpose of perfuming, modifying the skin appearance and/or correcting body odors and/or protecting them or keeping them in good condition.

In a variant of the invention, the cosmetic composition can also incorporate active ingredients of lipophilic and hydrophilic nature which, although they do not have therapeutic effect, they have properties as cosmetic agent. The following can be mentioned among the active ingredients which can be incorporated in the nanocapsule systems: emollients, preservatives, fragrance substances, anti-acne agents, antifungal agents, antioxidants, deodorants, antiperspirants, antidandruff agents, depigmentation agents, antiseborrheic agents, dyes, bronzing lotions, UV light absorbers, enzymes, fragrance substances, among other.

As has been described above, there is the possibility that the nanocapsule systems described in the present invention incorporate more than one active ingredient which may be dissolved in the same solution or separately, this depending on the nature of the molecules to be incorporated, preventing the existence of any other type of interaction, either chemical or physical interaction, among them.

To better understand the features and advantages of the present invention, a series of examples will be referred to below which explicatively complete the previous description without being limited in any manner to the same.

### Examples

A series of abbreviations will be used for explaining the following examples:
PArg = polyarginine
PArg nanocapsules = This term is used due for simplicity in the examples and the drawings to refer to the nanosystems the nanocapsules of which comprise PArg, Miglyol® 812 and lecithin. PArg-PEG Nanocapsule = This term is used for simplicity in the examples and the drawings to refer to the nanosystems the nanocapsules of which comprise PArg, Miglyol® 812, lecithin and PEG stearate.
DCX = docetaxel
pDNA = plasmid deoxyribonucleic acid

The PArg salt used in the following examples was the poly-L-arginine hydrochloride having weight molecular between 5000 and 15000 Da.

### Example 1

### Evaluation of the physiochemical characteristics of the PArg nanocapsules and PArg-PEG nanocapsules according to the amount of polymer

Example 1.1. Nanocapsules made up of PArg coated oily cores were prepared according to the method for diffusing solvent in one step:
a) an aqueous solution of PArg hydrochloride (10 ml) was prepared in which 1 to 30 mg of PArg is dissolved;
b) an oily phase formed by a ethanol/acetone (0.25:4.7 ml) solution of lecithin (20 mg) was prepared to which 62.5 µl of Miglyol® 812 is added. Optionally, PEG stearate (48-96 mg) can be incorporated in this step to give rise to the 1 and 2% w/v PArg-PEG nanocapsules respectively;
c) the solutions resulting from steps a) and b) were mixed under magnetic stirring for 5 minutes, the nanocapsules being spontaneously obtained;
d) the organic solvents were evaporated to a constant volume.

When in the embodiment of this method the nanocapsules were made up of 2.5 and 5 mg of PArg hydrochloride, the PArg nanocapsules at 3 and 6% w/w, respectively, were obtained.

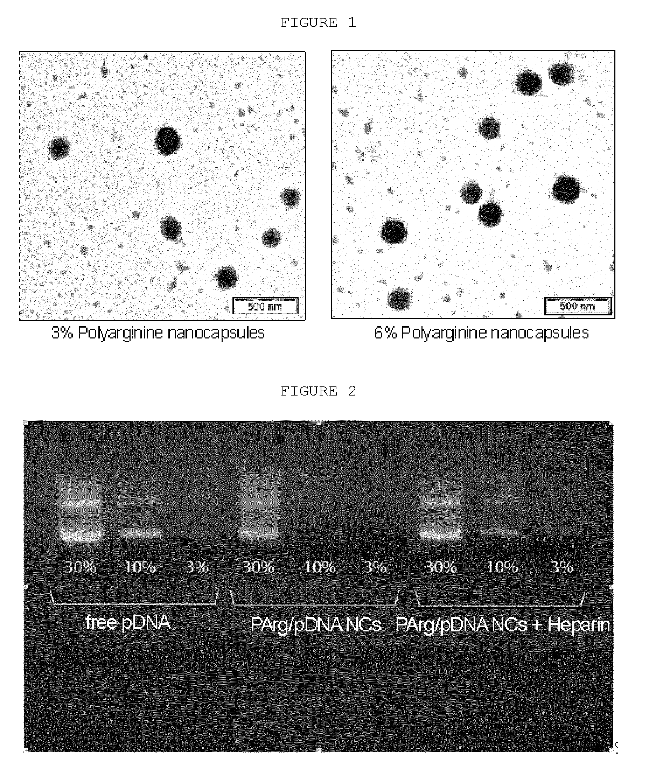

Once prepared, their mean diameter, polydispersity index as well as their surface electric charge (zeta potential) was measured and photos of the nanocapsules were taken by means of transmission electron microscopy. Table 1 and Figure 1 show the values obtained from the mentioned parameters according to the amount of PArg in the aqueous solution of step a).

**Table 1**

| Formulation | PArg step a) (mg) | Size (nm) | PI | Zeta potential (mV) |
|---|---|---|---|---|
| PArg NCs | 1 | 167 ± 1 | 0.01 | +35 ± 1 |
| PArg-PEG NCs 1% w/v | 1 | 150 ± 2 | 0.09 | +25 ± 1 |
| PArg-PEG NCs 2% w/v | 1 | 117 ± 1 | 0.12 | +27 ± 1 |
| PArg NCs | 2.5 | 137 ± 11 | 0.13 | + 50 ± 6 |
| PArg NCs | 5 | 145 ± 13 | 0.10 | + 53 ± 6 |
| PArg NCs | 25 | 120 ± 2 | 0.11 | +61 ± 1 |
| PArg NCs | 30 | 199 ± 1 | 0.54 | +56 ± 2 |
| PArg NCs-PEG 1% w/v | 30 | 122 ± 4 | 0.37 | +43 ± 2 |
| PArg NCs-PEG 2% w/v | 30 | 111 ± 2 | 0.11 | +41 ± 3 |

Example 1.2. Nanocapsules made up of PArg coated oily cores were prepared according to the method for diffusing solvent in two steps:
i) an oily phase formed by an ethanol/acetone (0.25:4.7 ml) solution of lecithin (20 mg) was prepared to which 62.5 µl of Miglyol® 812 is added. Optionally PEG stearate (48-96 mg) can be incorporated to give rise to the 1 and 2% w/v PArg-PEG nanocapsules respectively;
ii) the solution obtained in step i) is added on 10 ml of bidistilled water under magnetic stirring being maintained for 5 minutes, the nanoemulsion is thus spontaneously obtained;
iii) the organic solvents were evaporated to a constant volume;
iv) the nanoemulsion obtained in step iii) was recoated by means of an incubation process with an aqueous solution (1 ml) formed by 0.1 to 60 mg of PArg, in a 4:1 (nanoemulsion:solution of PArg) ratio, the coating being immediately produced regardless of the temperature.

Once prepared, their mean diameter, polydispersity index as well as their surface electric charge (zeta potential) was measured. Table 2 show the values obtained from the mentioned parameters according to the amount of PArg in step iv).

When in the embodiment of this method the nanocapsules were made up of 2.5 and 5 mg of PArg hydrochloride, the PArg nanocapsules at 3 and 6% w/w, respectively, were obtained.

**Table 2**

| Formulation | PArg step iv (mg) | Size (nm) | PI | Zeta potential (mV) |
|---|---|---|---|---|
| NE | - | 135 ± 4 | 0.16 | -59 ± 2 |
| NE-PEG 1% w/v | - | 119 ± 3 | 0.12 | -24 ± 3 |
| NE-PEG 2% w/v | - | 140 ± 2 | 0.08 | -12 ± 2 |
| PArg NCs | 0.05 | 156 ± 9 | 0.15 | -52 ± 2 |
| PArg NCs-PEG 1% w/v | 0.05 | 108 ± 3 | 0.11 | -8 ± 5 |
| PArg NCs-PEG 2% w/v | 0.05 | 135 ± 2 | 0.08 | +3 ± 1 |
| PArg NCs | 0.6 | 165 ± 3 | 0.16 | +44 ± 2 |
| PArg NCs | 5 | 158 ± 2 | 0.12 | +54 ± 3 |
| PArg NCs | 10 | 161 ± 1 | 0.16 | +55 ± 1 |
| PArg NCs | 30 | 334 ± 4 | 0.42 | +54 ± 1 |
| PArg-PEG NCs 1% w/v | 30 | 120 ± 1 | 0.16 | +28 ± 2 |
| PArg-PEG NCs 2% w/v | 30 | 136 ± 1 | 0.08 | +28 ± 1 |

Example 1.3. Nanocapsules made up of PArg coated oily cores were prepared according to the method of sonication:
i) an oily phase formed by a solution of lecithin (20 mg) in dichloromethane (1 ml) was prepared to which 62.5 µl of Miglyol® 812 is added;
ii) the solution obtained in step i) was added on 2 ml of bidistilled water and was sonicated for 1 minute;
iii) the emulsion obtained was diluted with water (dilution 1:10);
iv) the organic solvents were evaporated to a constant volume to form a nanoemulsion; and
v) the nanoemulsion obtained in step iv) was recoated by means of an incubation process with an aqueous solution (1 ml) formed by 0.1 to 60 mg of PArg hydrochloride, in a 4:1 (nanoemulsion:solution of PArg) ratio, the coating being immediately produced regardless of the temperature.

Once prepared, their mean diameter, polydispersity index as well as their surface electric charge (zeta potential) was measured. Table 3 show the values obtained from the mentioned parameters according to the amount of PArg in step v).

**Table 3**

| Formulation | PArg step v) (mg) | Size (nm) | PI | Zeta potential (mV) |
|---|---|---|---|---|
| NE | - | 192 ± 9 | 0.09 | -50 ± 7 |
| PArg NCs | 0.05 | 238 ± 14 | 0.18 | -53 ± 3 |
| PArg NCs | 0.6 | 212 ± 10 | 0.17 | +49 ± 1 |
| PArg NCs | 5 | 224 ± 6 | 0.13 | +60 ± 1 |
| PArg NCs | 10 | 204 ± 2 | 0.14 | +65 ± 1 |
| PArg NCs | 30 | 248 ± 4 | 0.19 | +60 ± 1 |

Example 1.4. Nanocapsules made up of PArg coated oily cores were prepared according to the method of homogenization:
i) an oily phase formed by a solution of lecithin (20 mg) in dichloromethane (1 ml) was prepared to which 62.5 µl of Miglyol® 812 is added;
ii) the solution obtained in step i) was added on 2 ml of bidistilled water and was homogenized at 16,000 rpm for 5 minutes and then at 19,000 rpm for another 5 minutes;
iii) the emulsion obtained was diluted with water (dilution 1:10) and was homogenized for 3 minutes at 22,000 rpm;
iv) the organic solvents were evaporated to a constant volume to form a nanoemulsion; and
v) the nanoemulsion obtained in step iv) was recoated by means of an incubation process with an aqueous solution (1 ml) formed by 0.1 to 60 mg of PArg hydrochloride, in a 4:1 (nanoemulsion:solution of PArg) ratio, the coating being immediately produced regardless of the temperature.

Once prepared, their mean diameter, polydispersity index as well as their surface electric charge (zeta potential) was measured. Table 4 show the values obtained from the mentioned parameters according to the amount of PArg in step v).

**Table 4**

| Formulation | PArg step v) (mg) | Size (nm) | PI | Zeta potential (mV) |
|---|---|---|---|---|
| NE | - | 230 ± 7 | 0.19 | -53 ± 5 |
| PArg NCs | 0.05 | 343 ± 37 | 0.26 | -41 ± 3 |
| PArg NCs | 0.6 | 292 ± 5 | 0.24 | +48 ± 2 |
| PArg NCs | 5 | 253 ± 36 | 0.19 | +60 ± 2 |
| PArg NCs | 10 | 228 ± 3 | 0.19 | +64 ± 1 |
| PArg NCs | 30 | 248 ± 4 | 0.45 | +63 ± 5 |

### Example 2

### Evaluation of the genetic material encapsulating capacity in PArg nanocapsules

PArg nanocapsules in the form of hydrochloride and an oily core formed by lecithin and Miglyol® 812 were prepared according to the method described above in Example 1.1. The association of a negatively charged hydrophilic macromolecule was performed taking pDNA as model. The active ingredient was incorporated by means of adsorbing the genetic material dissolved in an aqueous phase on a suspension of previously isolated PArg nanocapsules.

The association of pDNA on the PArg nanocapsules can be performed at different genetic material/nanocapsule load proportions. For the pDNA and the PArg used in the present study it was found that the optimum proportions were 3 and 10% (pDNA/PArg NC). Table 5 shows the data corresponding to the mean diameter, polydispersity index and surface charge of the nanocapsules incorporating pDNA.

**Table 5**

| | Size (nm) | PI | Zeta potential (mV) |
|---|---|---|---|
| PArg NC | 123 ± 7 | 0.20 | +56 ±2 |
| 3% pDNA/PArg NC | 129 ± 4 | 0.22 | +47 ± 2 |
| 10% pDNA/PArg NC | 136 ± 9 | 0.16 | +31 ± 6 |

The association efficiency (evaluating the free pDNA by means of electrophoresis complete association of the pDNA to the nanocapsules was observed, the presence of free plasmid not being detected in the gel. Furthermore, it was observed that said interaction was very strong, the addition of high concentrations of heparin (15 mg/ml) being necessary to move the pDNA (Figure 2). For the proportion of 30% a significant association was observed although the presence of free plasmid was also detected in the gel. Therefore the proportion of 3% was selected for the rest of the studies with pDNA.

The potential of the PArg nanocapsules as systems for carrying pDNA was then evaluated in comparison with the pDNA/PArg complexes described above in the literature (Rudolph et al. J. Biol. Chem. (2003) 278(13):11411). To that end a series of pDNA/PArg complexes having 5:1-1:5 ratios by weight was prepared. The values of mean diameter, polydispersity index as well as of surface electric charge (zeta potential) of the complexes are shown in Table 6.

**Table 6**

| Formulation | Size (nm) | PI | Zeta potential (mV) |
|---|---|---|---|
| Complexes 5:1 | 176 ± 63 | 0.32 | -11 ± 2 |
| Complexes 3:1 | 101 ± 42 | 0.24 | -12 ± 2 |
| Complexes 1:1 | 79 ± 13 | 0.21 | +19 ± 3 |
| Complexes 1:3 | 150 ± 77 | 0.31 | +14 ± 1 |
| Complexes 1:5 | 132 ± 20 | 0.28 | +9 ± 1 |

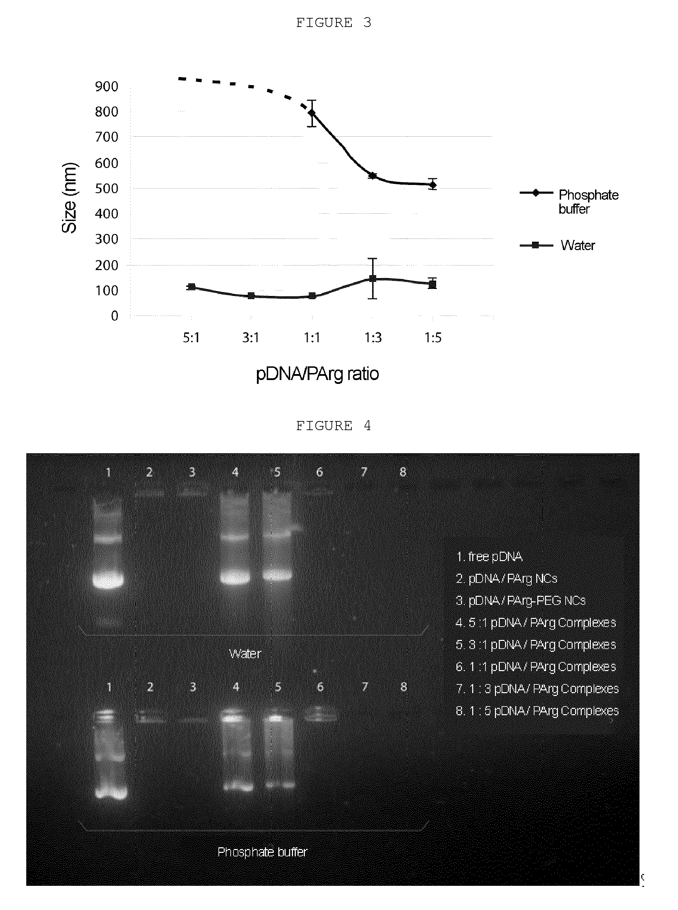

As observed in Figure 3, the pDNA/PArg complexes have sizes greater than 200 nm when they are suspended in water. However, after diluting them in phosphate buffer the massive aggregation of the complexes is produced, which clearly shows their instability in biological fluids. Contrarily, the pDNA/PArg nanocapsules and also those of pDNA/PArg-PEG have a much more favorable behavior: the pDNA/PArg-PEG nanocapsules remained stable after their dilution in phosphate buffer and those of pDNA/PArg underwent an initial size increase of 200 nm, but they were in no case object of massive aggregation.

The possible premature release of DNA from the nanocapsules after their incubation in phosphate buffer was also evaluated. The results of gel electrophoresis shown in Figure 4 show that both the PArg nanocapsules and those of PArg-PEG efficiently associate the pDNA and they do not release it after incubation in phosphate buffer.

### Example 3

### Evaluation of the hydrophobic docetaxel drug encapsulating capacity in PArg nanocapsules

PArg nanocapsules in the form of hydrochloride and an oily core formed by lecithin and Miglyol® 812 were prepared. The incorporation of a hydrophobic drug, taking for that purpose docetaxel, an anti-tumor agent practically insoluble in water was performed. The method of manufacturing corresponds to the method described above in Example 1.1., with a modification, since a small aliquot from a stock solution of the active ingredient in ethanol (1-100 mg/ml) is incorporated to the oily phase. Then said oily phase is added on an aqueous phase containing the PArg, the docetaxel encapsulating PArg nanocapsules with proportions by weight of docetaxel/PArg nanocapsules of up to 4% being formed.

Once the nanocapsules were prepared according to the method of the invention the encapsulation efficiency (evaluating the free drug by means of high resolution liquid chromatography with λ=227 nm) was determined, an encapsulation efficiency of 74% being obtained. The parameters mean particle diameter, polydispersity index and zeta potential (Table 7) were also measured.

**Table 7**

| | Size (nm) | PI | Zeta potential (mV) |
|---|---|---|---|
| PArg NC | 145 ± 13 | 0.1 | +53 ± 6 |
| DCX PArg NC | 170 ± 10 | 0.1 | +56 ± 6 |

### Example 4

### Release of the docetaxel drug from the PArg nanocapsules

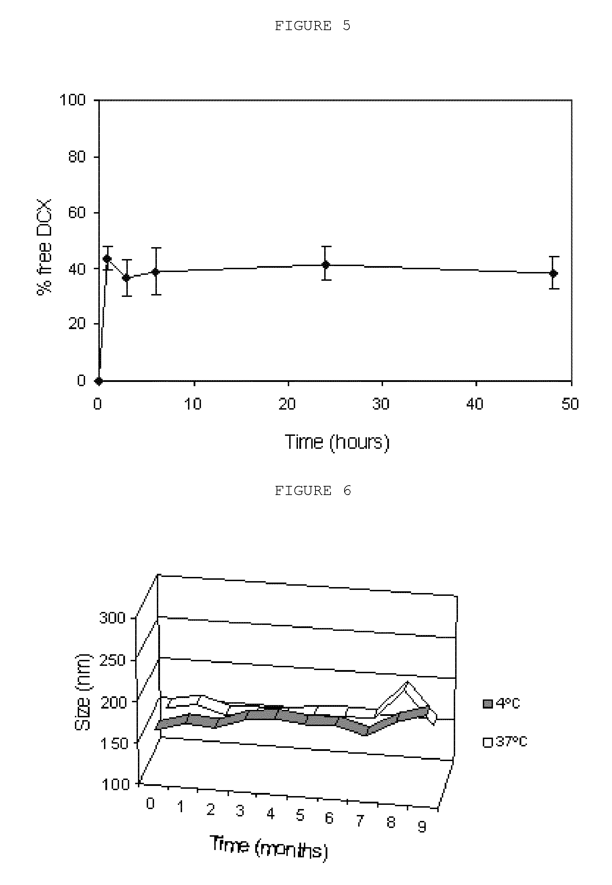

Hydrophobic docetaxel drug-encapsulating PArg nanocapsules were made following the method described in Example 3. The nanocapsules were diluted in acetate buffer (pH 5, low ionic force) and incubated in this medium under horizontal stirring (100 rpm) at 37°C. At different times (1, 3, 6, 24 and 48 hours), samples were taken from the incubation media and the nanocapsules in suspension were isolated by means of ultrafiltration. Finally the fraction of drug released was indirectly assessed by quantifying the amount of drug retained in the nanocapsules. The docetaxel was quantified as described in Example 3. The drug release profile for the PArg nanocapsules is shown in Figure 5.

### Example 5

### Evaluation of the particle size of the PArg nanocapsule formulation during the storage thereof

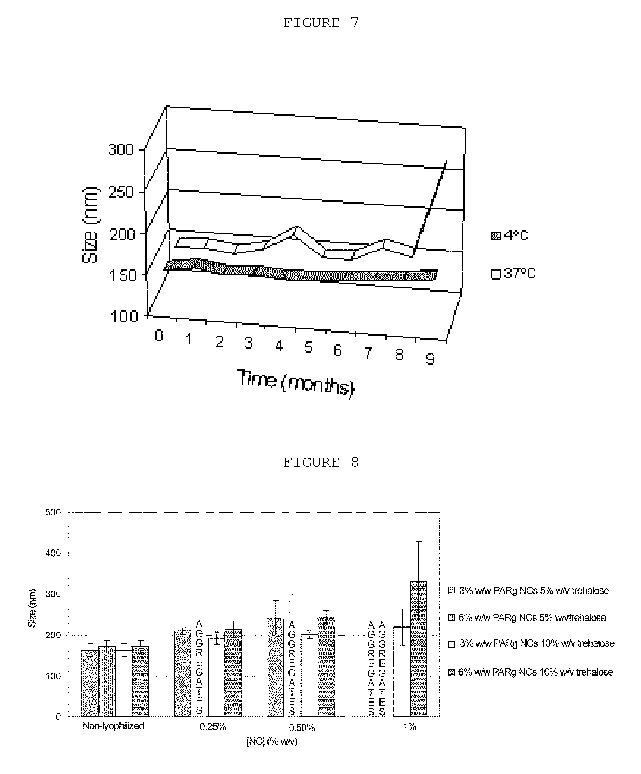

PArg nanocapsules in the form of hydrochloride, an oily core formed by lecithin and Miglyol® 812 were prepared according to the method described above. Particle size was measured for nine months in order to obtain information about the evolution of the system over time. The effect of the storage temperature (4 and 37°C) on the stability of the nanocapsules was also evaluated. The results presented in Figures 6 and 7 show the small variability of the size of the PArg nanocapsules at 3 and 6% w/w, respectively, during storage.

### Example 6

### Evaluation of the trehalose cryoprotective effect on the particle size of the PArg nanocapsules after the lyophilization process

PArg nanocapsules in the form of hydrochloride, an oily core formed by lecithin and Miglyol® 812 were prepared according to the method described above. The effect which the trehalose cryoprotectant agent has was evaluated during the PArg nanocapsule lyophilization process and in the subsequent recovery of the particle size after the resuspension by assaying two concentrations of trehalose, 5 and 10% w/v. Likewise, the influence of the concentration of nanocapsules (0.25, 0.5 and 1% w/v) in the suspension to be lyophilized was evaluated as the effect of the concentration of PArg in the nanosystem (3 and 6% w/w). The results of Figure 8 show the particle size of the lyophilized PArg nanocapsules after the resuspension. It is observed that the high concentrations of cryoprotectant as well as the low PArg ratio in the sample preserve the size of the nanocapsules.

### Example 7

### Study of the capacity to inhibit cell proliferation of the PArg nanocapsules

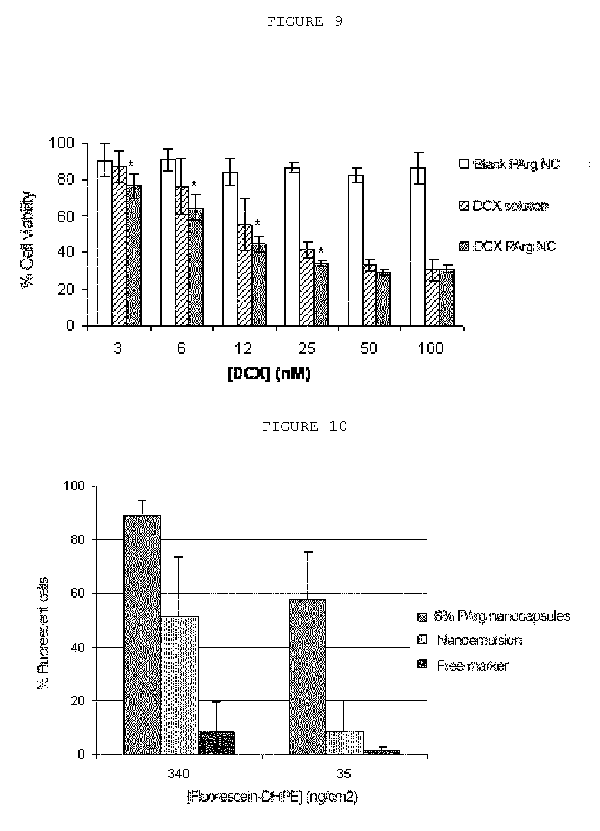

In order to evaluate the potential of the PArg nanocapsules encapsulating the docetaxel cytostatic molecule to inhibit the cell proliferation of the NCI-H460 lung cancer cell line the nanocapsules were prepared according to the method described in Example 3. The results shown in Figure 9 show the great proliferation inhibition capacity of the nanocapsules encapsulating docetaxel in comparison with the free molecule. In addition, the uncharged PArg nanocapsule formulation did not show any toxicity in the assayed range of concentrations. The IC50 values, concentration at which the proliferation of 50% of the population is inhibited, were calculated by means of the Graph Pad Prism 2.1 program (Graph Pad Software). Thus, the docetaxel solution had an IC50 value of 13.35 nM whereas for the PArg nanocapsule formulation encapsulating docetaxel was 3.28 nM, the nanoencapsulation being 4 times more effective in inhibiting cell proliferation.

### Example 8

### Study of the cellular uptake of the PArg nanocapsules

In order to evaluate the potential of the PArg as a cell entry promoter, the interaction of the PArg nanocapsules with the NCI-H460 cell line in comparison with the nanoemulsion was studied. A hydrophobic fluorescent molecule (fluorescein-DHPE) was incorporated previously in the PArg nanocapsule formulation by means of dissolving the marker in ethanol and then incorporating it in the oily phase. Figure 10 shows the values obtained from cell entry of the PArg nanocapsules in comparison with the nanoemulsion and the free fluorescent label. The results show high cellular uptake of the nanocapsule formulation suggesting that the PArg coat is essential to facilitate the entry process of the nanosystems into the cells.

### Example 9

### Study of the cellular uptake of the PArg nanocapsules depending on the amount of PArg in the nanosystem

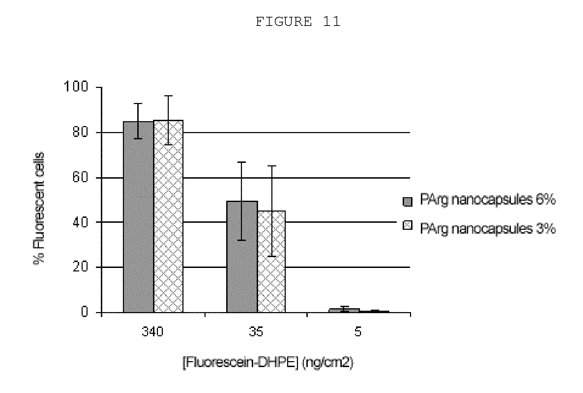

In order to evaluate if the higher amount of PArg in the nanocapsules favors cell interaction and subsequent internalization, the cellular uptake of two PArg nanocapsule formulations with the NCI-H460 cell line was studied. A hydrophobic fluorescent molecule (fluorescein-DHPE) was incorporated previously in the PArg nanocapsule formulation by means of dissolving the marker in ethanol and then incorporating it in the oily phase. Figure 11 shows the values obtained from cell entry of the PArg nanocapsule formulations at 3 and 6% w/w. The results show that both systems have a very similar behavior with respect to the cellular uptake.

## Claims

1. A system for administering active ingredients comprising nanocapsules with a diameter less than 1µm comprising a polyarginine salt, a negative phospholipid and an oil.

2. The system according to claim 1 additionally comprising one or more active ingredients.

3. The system according to any of the preceding claims, additionally comprising a hydrophobic auxiliary ingredient; preferably a polyoxyethylene derivative; and more preferably polyethylene glycol stearate.

4. The system according to any of the preceding claims, wherein the polyarginine salt is selected from hydrochloride, hydrobromide, acetate and sulfate; said salt is preferably hydrochloride.

5. The system according to any of the preceding claims, wherein the negative phospholipid is selected from lecithin, phosphatidylglycerol, phosphatidylserine, phosphatidylinositol, diphosphatidylglycerol and phosphatidic acid; said negative phospholipid is preferably lecithin.

6. The system according to any of the preceding claims, wherein the oil is selected from peanut oil, cotton oil, olive oil, castor oil, soybean oil, safflower oil, palm oil; vitamin E, isopropyl myristate, squalene, Mygliol®, Labrafil®, Labrafac®, Peceol® and Maisine®; said oil is preferably Mygliol®.

7. The system according to any of the preceding claims, comprising an active ingredient selected from peptides, proteins, lipid or lipophilic compounds, saccharide compounds, compounds of nucleic acids and nucleotides or combinations thereof.

8. The system according to claim 7, wherein the active ingredient is docetaxel.

9. The system according to claim 7, wherein the active ingredient is selected from an oligonucleotide, interference RNA, a DNA plasmid or a polynucleotide; the active ingredient is preferably a DNA plasmid.

10. The system according to any of the preceding claims, **characterized in that** it is in lyophilized form.

11. The method for obtaining the system defined in any of claims 1 to 10, which comprises:
a)preparing an aqueous solution of a polyarginine salt;
b)preparing an organic solution of a negative phospholipid and an oil;
c)mixing the solutions prepared in steps a) and b) under stirring, the systems being spontaneously obtained; and
d)optionally, completely or partially evaporating the organic solvents from the mixture obtained in the preceding step to a constant volume.

12. The method according to claim 11, which further comprises adding an active ingredient.

13. The method for obtaining the system defined in any of claims 1 to 10, which comprises coating a nanoemulsion made up of at least a negative phospholipid, an oil and an aqueous phase, by means of an incubation process with an aqueous solution of a polyarginine salt.

14. The method according to claim 13, wherein the incubation process comprises mixing the nanoemulsion with an aqueous solution of polyarginine, preferably at a 4:1 ratio.

15. The method according to any of claims 13 to 14, which further comprises adding an active ingredient.

16. The method according to any of claims 11 to 15, which next comprises a lyophilization step of the obtained systems in the presence of cryoprotectants.

17. The method according to the preceding claim, which next comprises a step for regenerating the lyophilized systems.

18. A pharmaceutical composition comprising the system defined in any of claims 1 to 10.

19. The pharmaceutical composition according to the preceding claim, wherein said composition is for administering topically, parenterally or through mucosae.

20. A cosmetic composition comprising the system defined in any of claims 1 to 10 together with one or more cosmetically acceptable excipients.

21. Use of a system as it has been defined in any of claims 1 to 10 in the preparation of a drug.

22. Use of a system as it has been defined in any of claims 1 to 10 in the preparation of a drug for treating cancer.
